Europäisches Patentamt

European Patent Office

Office européen des brevets

(19)

(11) Numéro de publication: **0 229 577 B1**

## FASCICULE DE BREVET EUROPEEN

(12)

(45) Date de publication de fascicule du brevet:
**24.04.91**

(51) Int. Cl.⁵: **A61F 13/06**

(21) Numéro de dépôt: **86420300.5**

(22) Date de dépôt: **11.12.86**

(54) **Genouillère de réadaptation fonctionnelle perfectionnée.**

(30) Priorité: **13.12.85 FR 8518811**

(43) Date de publication de la demande:
**22.07.87 Bulletin 87/30**

(45) Mention de la délivrance du brevet:
**24.04.91 Bulletin 91/17**

(84) Etats contractants désignés:
**AT BE CH DE ES GB GR IT LI LU NL SE**

(56) Documents cités:
**FR-A- 2 532 839**
**. FR-A- 2 570 595**
**US-A- 3 306 288**
**US-A- 3 451 232**
**US-A- 4 474 573**

(73) Titulaire: **Bertheas, Michel**
**1, rue Jean Baptiste Marcet**
**F-42170 St Just St Rambert(FR)**

(72) Inventeur: **Bertheas, Michel**
**1, rue Jean Baptiste Marcet**
**F-42170 St Just St Rambert(FR)**

(74) Mandataire: **Dupuis, François**
**Cabinet Laurent et Charras 3, place de**
**l'Hôtel-de-Ville**
**F-42000 Saint-Etienne(FR)**

EP 0 229 577 B1

Rank Xerox (UK) Business Services

## Description

L'invention concerne une genouillère de réadaptation fonctionnelle perfectionnée.

Les genouillères réalisées en tissu élastique ont pour fonction d'envelopper la jambe de part et d'autre du genou pour maintenir et protéger l'articulation. Des genouillères de ce type ont été décrites par exemple dans la demande de brevet français 82.15821 (FR-A-2532839) et dans la demande de brevet européen 84.420042.8 (EP-A-0154758) appartenant au déposant.

Les genouillères précitées sont couramment exploitées et répondent de manière satisfaisante aux besoins et attentes de leurs utilisateurs.

Lors d'un usage intensif et durable par l'utilisateur, on a constaté que la genouillère quelque soit son type et ses caractéristiques engendrait une certaine gêne et créait des échauffements désagréables et douloureux sur la partie de pliage de genou, ceci par les contacts et les frottements de la contexture du tissu de la partie arrière de la genouillère avec la peau, inhérents aux mouvements d'articulation de la jambe.

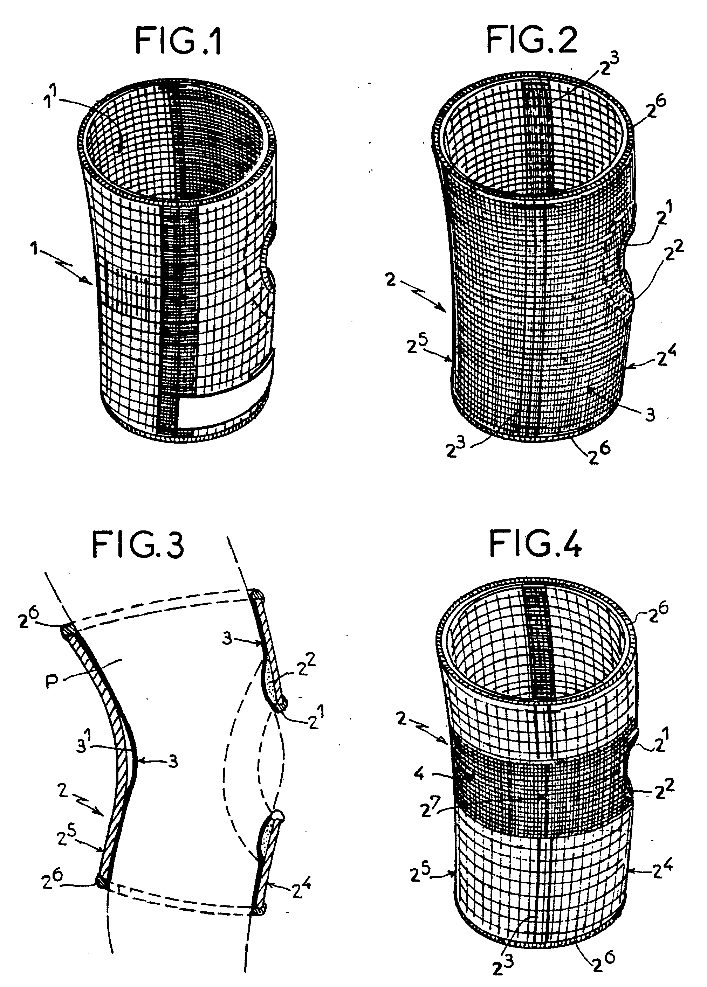

La figure 1 du dessin illustre une genouillère (1) selon l'art antérieur précité mettant en valeur la zone de frottement (1¹). Pour éviter de telles gênes, les utilisateurs ont l'habitude de mettre du talc sur les zones sujettes à inflammation. Cela n'est cependant pas pratique et ne résout pas en soi le problème.

On connaît également une genouillère telle que décrite dans le brevet US 4.474.573. Celle-ci est réalisée avec une double paroi correspondant à une sorte de matelassage comprenant une étoffe extérieure tricotée associée à une mousse en élastomère. L'étoffe est agglomérée à la mousse par lamification de flamme ou agglomération adhésive et est ainsi étroitement combinée avec l'étoffe extérieure. Tout mouvement ou déformation de la paroi extérieure implique une déformation du matelassage. Une réalisation complexe de la genouillère à été proposée avec formation de panneaux en forme de losange situés dans la partie arrière de celle-ci.

La genouillère ainsi décrite ne répond pas de maniére satisfaisante au problème posé et ne supprime pas les micro-traumatismes, échauffements et irritations lors de la flexion du genou.

Le but recherché selon l'invention était donc de remédier aux inconvénients précités.

L'invention propose une solution simple allant à l'encontre des techniques proposées à ce jour, selon une mise en oeuvre simple, offrant un confort nouveau à l'utilisateur et évitant les inflammations sur les parties exposées.

Selon une première caractéristique, la genouillère de réadaptation fonctionnelle se présente sous la forme d'une enveloppe tubulaire tissée ou tricotée déformable dans un ou plusieurs sens et composée au moins partiellement de deux couches ou parois, caractérisée en ce qu'elle comprend une première paroi en forme d'une contexture extérieure faisant fonction de tissu de contention et qu'elle comprend sur tout ou partie de sa surface intérieure, une seconde paroi réalisée en matériau souple, de contexture fine formant peau, susceptible de s'appliquer par une face directement sur la peau de l'utilisateur, l'autre face étant en contact avec la contexture extérieure de la genouillère, ladite seconde paroi constituant un intermédiaire entre ladite contexture extérieure et la peau de l'utilisateur, permettant le glissement indépendant de la contexture extérieure par rapport à la seconde paroi.

Selon l'invention, l'intérêt de disposer une seconde paroi non solidaire du tissu de contention réside dans la nécessité de démultiplier les frictions sources d'irritatians cutanées dangereuses.

Cette deuxième paroi très fine et très souple est en contact direct avec la peau et par ses caractéristiques, elle s'adapte au mieux au relief anatomique du genou.

Ces caractéristiques et d'autres encore ressortiront bien de la description.

Pour fixer l'objet de l'invention illustré d'une manière non limitative aux figures des dessins où :

- la figure 1 illustre une genouillère classique selon l'art antérieur,
- la figure 2 illustre une genouillère selon l'invention après retournement laissant voir sa face intérieure,
- la figure 3 est une vue en coupe longitudinale illustrant la genouillère selon l'invention apposée sur le genou de l'utilisateur,
- la figure 4 est une vue en variante d'une réalisation de la genouillère.

La genouillère (2) est réalisée de manière classique par tissage ou tricotage avec une capacité élastique de déformation dans un ou plusieurs sens et se présente sous la forme d'une enveloppe tubulaire susceptible de s'appliquer et d'épouser étroitement la forme de la jambe d'une personne au niveau de son genou. Cette genouillère présente, par exemple et non limitativement, sur sa partie frontale, une ouverture ($2_1$) pour le passage du genou, entourée par l'intérieur d'un coussin annulaire ($2^2$) avec évidement dans la partie postérieure pour le passage du tendon rotulien.

Latéralement, de chaque côté, la genouillère comprend une ou plusieurs bandes ou gaines ($2_3$) de séparation de la partie frontale ($2^4$) et de la partie arrière ($2_5$) contribuant à la solidité et à l'élasticité de la genouillère. On pourrait prévoir que la genouillère se présente sous la forme d'une enveloppe tubulaire continue.

Selon l'invention et pour éviter que la contexture tissée ou tricotée de la partie arrière de la genouillère vienne en appui directement sur la jambe au niveau de l'endroit de pliure du genou, il est prévu un aménagement particulier de ladite partie arrière de la genouillère offrant un confort accru de l'utilisateur.

A cet effet, il est prévu que la genouillère présente sur tout ou partie de sa partie arrière, une paroi (3) complémentaire de contexture fine déformable formant une peau susceptible de s'appliquer directement sur la peau (P) de l'utilisateur. Cette paroi (3) réalisée en toute matière fibres naturelles, mousse ou autre, évite ainsi tout contact entre la contexture de la genouillère et la peau de l'utilisateur. Par ailleurs, c'est la face (3¹) de la membrane (3) en regard avec la contexture (2₅) qui joue le rôle d'appui et de contact avec celle-ci. De part leur nature de réalisation différente, la contexture de la genouillère vient avantageusement glisser sur la peau complémentaire hors des différents mouvements de la jambe, ceci sans aucun effet direct sur la peau de l'utilisateur.

Cette double paroi joue un rôle physiologique car elle colle étroitement à la peau tandis que les reliefs de la contexture de la genouillère glissent sans risque de blessures ou d'échauffements sur la paroi en regard.

Cette paroi de protection ou seconde peau peut recouvrir l'ensemble de la surface constitutive de la partie arrière de la genouillère. En variante, comme illustré figure 4, elle se présente sous la forme d'une simple bande (4) se situant par exemple dans la partie centrale de la genouillère entourant l'ouverture s'il y en a une autorisant le passage de la rotule.

Cette seconde paroi est fabriquée directement ou est rapportée lors de la réalisation de la genouillère et est fixée aux bordures extérieures (2⁶) et latérales (2₇) de manière appropriée bien connue de l'homme du métier. Dans le cas d'une genouillère simple, la double paroi peut être réalisée sur tout ou partie de sa surface intérieure.

En variante, la seconde paroi est réalisée sur la totalité de la genouillère, face avant, face arrière, en une ou plusieurs parties.

Les avantages ressortent bien de l'invention. On souligne sa simplicité de réalisation et le meilleur confort qu'elle procure à l'usage.

rois, caractérisée en ce qu'elle comprend une première paroi en forme d'une contexture extérieure faisant fonction de tissu de contention et qu'elle comprend sur tout ou partie de sa surface intérieure, une seconde parci (3) réalisée en matériau souple, de contexture fine formant peau, susceptible de s'appliquer par une face directement sur la peau (P) de l'utilisateur, l'autre face (3.1) étant en contact avec la contexture extérieure de la genouillère, ladite seconde paroi constituant un intermédiaire entre ladite contexture extérieure et la peau (P) de l'utilisateur, permettant le glissement indépendant de la contexture extérieure par rapport à la seconde paroi.

2. Genouillère selon la revendication 1, caractérisée en ce que l'enveloppe tubulaire de la genouillère est réalisée en continu, la seconde paroi (3) recouvrant tout ou partie de la surface intérieure de celle-ci.

3. Genouillère de réadaptation selon la revendication 1, caractérisée en ce que l'enveloppe tubulaire constitutive de la genouillère comprend une partie frontale (2.4.) et une partie arrière (2.5.) avec une ou plusieurs gaines ou bandes de séparation (2.3.), la seconde peau recouvrant tout ou partie de la surface intérieure de la partie arrière de la genouillère à l'endroit du pliage du genou.

4. Genouillère selon l'une quelconque des revendications 1, 2 et 3, caractérisée en ce que la seconde paroi de contexture fine est déformable pour s'appliquer et épouser directement le profil de la jambe à recouvrir.

5. Genouillère selon l'une quelconque des revendications 1, 2 et 3, caractérisée en ce que la seconde paroi est solidarisée directement ou d'une manière rapportée aux bordures extérieures et/ou latérales de la genouillère.

6. Genouillère selon la revendication 1, caractérisée en ce que la seconde peau (3) est réalisée en toute matière, fibres naturelles, mousse ou autre, de nature différente de la contexture tissée ou tricotée de l'enveloppe tubulaire.

**Revendications**

1. Genouillère de réadaptation fonctionnelle du type se présentant sous la forme d'une enveloppe tubulaire tissée ou tricotée déformable dans un ou plusieurs sens et composée au moins partiellement de deux couches ou pa-

**Claims**

1. Functional readaptation knee-pad of the type embodying the form of a woven or knitted tubular casing deformable in one or more directions, and consisting at least in part of two layers or walls, characterized in that said knee-pad includes a first wall in the form of an external contexture serving as a retention fabric, and includes on its entire inside surface or on a part thereof a second wall (3) made of soft material with a fine, skin-forming contexture, capable of being applied by one face directly on the skin (P) of the user, the other face (3.1) being in contact with the external contexture of the knee-pad, said second wall constituting an intermediate part between said external contexture and the skin (P) of the user, in order to permit the external contexture to be slipped over smoothly relative to the second wall.

2. Knee-pad as claimed in claim 1, characterized in that the tubular casing of the knee-pad is made continuously, the second wall (3) covering entirely or in part the inside surface of said casing.

3. Readaptation knee-pad as claimed in claim 1, characterized in that the tubular casing constitutive of the knee-pad comprises a front part (2.4.) and a rear part (2.5.) with one or more separation wrappings or strips (2.3.), the second skin covering entirely or in part the inside surface of the rear part of the knee-pad at the folding area of the knee.

4. Knee-pad as claimed in any one of claims 1, 2 and 3, characterized in that the second wall with a fine contexture is deformable in order to be applied on the leg to be covered and to fit directly the shape of this leg.

5. Knee-pad as claimed in any one of claims 1, 2 and 3, characterized in that the second wall, directly or in an inserted manner, is made fast or joined with the external and/or lateral edge portions of the knee-pad.

6. Knee-pad as claimed in claim 1, characterized in that the second skin (3) is made of any material, such as natural fibers, sponge material or the like, having a character different from the woven or knitted contexture of the tubular casing.

**Ansprüche**

1. Als eine rohrförmige, gewebte oder gestrickte, in eine oder mehrere Richtungen verformbare Umhüllung ausgeführter funktioneller Wiedererziehungkniegelenkhalter, der aus zweien Schichten oder Wandungen am wenigstens teilweise besteht, dadurch gekennzeichnet, dass dieser Kniegelenkhalter eine erste, in der Gestaltung eines äusserlichen Gefüges, als Festhaltengewebe wirkende Wandung aufweist, und auf seiner Innenfläche, ganz oder teilweise, eine zweite Wandung (3) auch aufweist, die aus einem Weichmaterial mit feinem, hautbilden Gefüge besteht, wobei diese zweite Wandung auf der Haut (p) des Benützers durch eine Fläche unmittelbar aufgelegt werden kann ; und dass die andere Fläche (3. 1) mit dem äusserlichen Gefüge des Kniegelenkhalters in Berührung ist, wobei die zweite Wandung, zwischen dem besagten äusserlichen Gefüge und der Haut (P) des Benützers, eine Zwischenschicht zum unabhängigen Durchgleiten des äusserlichen Gefüges in bezug zur zweiten Wandung bildet.

2. Kniegelenkhalter nach Anspruch 1, dadurch gekennzeichnet, dass die rohrförmige Umhüllung des Kniegelenkhalters kontinuierlich hergestellt wird, wobei die zweite Wandung (3) ganz oder teilweise die Innenfläche des Kniegelenkhalters überdeckt.

3. Wiedererziehungskniegelenkhalter nach Anspruch 1, dadurch gekennzeichnet, dass die den Kniegelenkhalter bildende, rohrförmige Umhüllung einen Stirnteil (2.4.) und einen Hinterteil (2.5.) mit einem oder mehreren Trennungsbändern- oder Streifen (2.3.) aufweist, wobei die zweite Haut ganz oder teilweise die Innenfläche des Hinterteils des Kniegelenkhalters an der Faltstelle des Knies überdeckt.

4. Kniegelenkhalter nach einem der Ansprüche 1, 2 und 3, dadurch gekennzeichnet, dass die zweite Wandung mit feinem Gefüge zum Auflegen auf dem zu überdeckenden Bein und zum unmittelbaren Anpassen auf dem Profil dieses Beins verformbar ist.

5. Kniegelenkhalter nach einem des Ansprüche 1, 2 und 3, dadurch gekennzeichnet, dass die zweite Wandung mit den äusserlichen und/oder seitlichen Umrandungen des Kniegelenkhalters unmittelbar oder in zusammengesztzter Weise festgemacht ist.

6. Kniegelenkhalter nach Anspruch 1, dadurch gekennzeichnet, dass die zweite Haut (3) aus beliebigen Werkstoff, Echtfasern, Schaummaterial oder desgleichen besteht, wobei die Art dieses Werkstoffs von der Art des gewebten oder gestrickten Gefüges der rohrförmigen Umhüllung unterschiedlich ist.

FIG.1

FIG.2

FIG.3

FIG.4

5